# EUROPEAN PATENT SPECIFICATION

(11) **EP 1 744 662 B1**
(45) Date of publication and mention of the grant of the patent: **24.08.2011**
(21) Application number: 05718755.1
(22) Date of filing: 19.04.2005
(51) Int. Cl.: A61B 5/00

(54) **PROTECTION MECHANISM FOR SPECTROSCOPIC ANALYSIS OF BIOLOGICAL TISSUE**
SCHUTZMECHANISMUS FÜR DIE SPEKTROSKOPISCHE ANALYSE VON BIOLOGISCHEM GEWEBE
MECANISME DE PROTECTION POUR ANALYSE SPECTROSCOPIQUE DE TISSU BIOLOGIQUE

(30) Priority: 06.05.2004 EP 04101962; 13.05.2004 EP 04102102
(43) Date of publication of application: 24.01.2007
(73) Proprietor: Koninklijke Philips Electronics N.V., 5621 BA Eindhoven (NL)
(72) Inventor: VAN BEEK, Michael Cornelis Philips Intellectual, 52066 Aachen (DE); RENSEN, Wouter Harry Jacinth Philips Intellectual, 52066 Aachen (DE); VAN DER VOORT, Marjolein c/o Philips Intellectual, 52066 Aachen (DE)
(74) Representative: Roche, Denis
(86) International application number: PCT/IB2005/051266
(87) International publication number: WO 2005/107578

(56) References cited:
- WO-A-00/25662
- WO-A-2004/042382
- US-A- 4 267 844

## Description

The present invention relates to the field of spectroscopic analysis of biological tissue and in particular to protection mechanisms for in vivo examination of biological tissue.

Usage of optical spectroscopic techniques for analytical purposes is as such known from the prior art. WO 02/057758 A1 and WO 02/057759 A1 show spectroscopic analysis apparatuses for in vivo non-invasive spectroscopic analysis of the composition of blood flowing through a capillary vessel underneath the skin of a patient. The position of the capillary vessel is determined by a monitoring system in order to identify a volume of interest to which an excitation beam for the spectroscopic analysis has to be directed. Preferably, the imaging and the spectral analysis of the volume of interest is performed simultaneously. Making use of simultaneous monitoring and spectral analyzing allows to increase the signal to noise ratio or the signal to background ratio of a detectable spectroscopic signal. In principle, any monitoring method providing a sufficient visualization of a capillary vessel can be applied.

A spectroscopic analysis makes for example use of Raman spectroscopy to measure the concentration of analytes in the blood. Typically, for Raman spectroscopy, a near infrared (NIR) laser is used with an output power of about 200mW. Since light in the near infrared range is not visible but can create serious damage in the human eye, medical examination systems making use of high power infrared radiation, such as e.g. a non-invasive blood analysis system, have to be equipped with safety precautions to prevent unintended exposure of sensitive body parts of a patient.

United States patent no. 4,267,844 describes an electro-optical medical instrument for measuring the presence of bilirubin in skin tissue. The electro-optical instrument comprises a holder which provides means for grasping the instrument and for pressing a light outlet and a light intake against the forehead of a newborn. At this moment, the holder is shifted downward with respect to the housing of the instrument against a predetermined biasing force. When the holder is shifted a predetermined distance with respect to the housing, a trigger switch is automatically closed for activation of the xenon flash tube so that tube releases a flash of light for measuring purposes. Thus, the force of pressuring the skin provides timing means for determining the firing of the flash.

The holder is upward and downward slidable relative to the housing. The holder is normally biased toward the upper side of the housing by a spring. If the housing, held by the holder, is pressed against the skin of a newborn baby, the holder will be moved against the force of the spring, with the lower side of the housing kept in contact with the skin. When holder is shifted the predetermined value relative to the housing, a projection of the holder pushes a pair of contacts forming said switch to a closed position, Thus, the switch is always closed when the pressure of the lower side of the housing on the skin reaches a predetermined value for acting the flash tube to emit a measurement flash light source.

Chinese patent application CN 1 983 252 A teaches an apparatus for non-invasive measurement of living body characteristics by photo acoustics. Said apparatus may comprise a touch sensor which detects the degree of contact of a measurement site and a body interface. The touch sensor may be used to control the measurement protocol. The touch sensor may be a device that measures pressure or electrical resistance, for example a mechanical actuator or piezoelectric actuator.

The European patent application EP1 080 683 A2 discloses a laser diode optical transducer assembly for non-invasive spectrophotometric blood oxygenation monitoring. This assembly also includes a light emitting diode (LED) safety device which is operable to turn the laser diodes off in the event that the assembly accidentally becomes detached from the patient's skin. This safety light emitting diode serves as a part of a laser safety interlock minimizing the possibility of laser light exposure to personnel using such a near infrared spectroscopy system.

The purpose of the laser safety interlock is to inhibit laser diode pulsing in case the near infrared spectroscopy probe is not securely attached to the subject. The LED will operate prior to the initiation of laser diode pulsing. LED light levels are monitored by a photo diode to detect secure near infrared spectroscopy probe attachment. Accidental probe detachment will automatically shut down the laser diodes. Low LED detected light levels would indicate that the laser light potentially is radiating in free space, or is obstructed. High LED detected light levels would indicate that the assembly is loose, by assuming that the light is reflecting off the skin or an object to the photo diode, without passing through biological tissue.

The laser safety interlock disclosed in EP 1 080 683 A2 makes particular use of a safety LED and a photo diode monitoring the light levels of the safety LED. Hence, such a near infrared spectroscopic assembly requires at least two additional components that are particularly designed for realizing a laser safety interlock. Moreover, the proposed laser safety interlock is based on a light emitting and monitoring technique. As a consequence, its functionality strongly depends on the reflectivity of the attached subject.

Problems may for example arise when the near infrared spectroscopy assembly is attached to a variety of different subjects featuring different surface conditions with varying reflectivity, such as e.g. differently colored skin of people belonging to different ethnic groups.

Another disadvantage of such a laser safety interlock arises when the near infrared spectroscopic assembly should be universally applied to different regions of a body featuring a different reflectivity such as e.g. the lower arm, ear lobes, inner cheek, tongue or nostrils.

The present invention therefore aims to provide a universal protection mechanism for a spectroscopic system for determining a property of biological tissue in a volume of interest of a patient.

The invention provides a spectroscopic system for determining of a property of a biological structure in a volume of interest. Preferably, the spectroscopic system is designed for in vivo and non-invasive analysis of biological tissue or fluid. The biological structure refers to various specific samples of tissue, e.g. underneath the surface of the skin, such as a particular blood vessel. Moreover, it may also refer to biological structures that are located at the surface of the skin, such as hair or particular glands. For example, a property of blood flowing through a blood vessel underneath the surface of the skin of a patient can be sufficiently determined by means of the non-invasive spectroscopic analysis.

The spectroscopic system has a measurement head for directing an excitation beam into the volume of interest and at least one detector element for determining if the measurement head is in a measurement position. Furthermore, the spectroscopic system comprises means for switching the excitation beam in response of an output from the at least one detector element. The measurement position refers to a relative position between the measurement head and the surface of the skin of the patient. Depending on the specific embodiment of the invention, the measurement head is in a measurement position either when the measurement head is in direct mechanical contact with the surface of the skin or when the measurement head is not in direct contact with the skin but is located in close proximity to the surface of the skin.

At least one detector element is adapted to produce an output indicating whether the measurement head is in a measurement position. In principle, the output of the at least one detector element can be any arbitrary signal, such as e.g. an electrical signal, a magnetic signal, a pneumatic signal, an optical signal, an acoustic signal or even a simple mechanical movement.

Also, the means for switching the excitation beam can be implemented in a plurality of different ways by e.g. a mechanical shutter, an electrically driven shutter, an acousto-optic modulator (AOM) or similar devices being adapted to intercept the propagation of the excitation beam. Alternatively, the means for switching the excitation beam can be implemented as a simple switch for switching off the power of the light source generating the excitation beam.

According to the invention, the at least one detector element is implemented as a pressure sensor being adapted to generate an output being indicative of a contact pressure between the measurement head of the spectroscopic system and the surface of the skin. A mechanism making use of a pressure sensor as detector element and means for switching off the excitation beam in response of a signal provided by the pressure sensor can effectively prohibit, that the high intensity excitation beam is accidentally released into free space. Only when the pressure sensor detects a predefined contact pressure a corresponding output signal is provided to the means for switching off the excitation beam.

The means for switching off the excitation beam are in turn adapted to release the excitation beam only if the provided pressure signal indicates that the measurement head is in mechanical contact with the skin of the patient. Moreover, by making use of such a pressure sensor, exposure of the skin by the excitation beam can also effectively be prohibited if the contact pressure exceeds a maximum allowable value. For example, when the contact pressure is extremely large, the skin of the patient and in particular the biological structure within the volume of interest may become subject to mechanical deformation thus falsifying the result of the spectroscopic analysis.

According to the invention, the at least one detector element comprises two electrodes. Furthermore, the at least one detector element is adapted to generate an electric signal being indicative of the electric resistance between the two electrodes. When the measurement head and in particular the two electrodes of the detector element of the measurement head are in mechanical contact with the surface of the skin of the patient, the two electrodes sufficiently allow to determine the electric resistance of the surface of the skin. When the electric resistance between the two electrodes of the at least one sensor element is within a predefined range, thus indicating that the detector element is in mechanical contact with the surface of the skin of the patient, the means for switching of the excitation beam are adapted to release the excitation beam into the volume of interest. In this particular embodiment, the invention makes use of the electric properties of the surface of the skin. It can effectively be prevented, that high power radiation is released from the measurement head when the detector element is surrounded by material featuring a substantially different electric resistance than the surface of a skin, like e.g. air or an electrical conductor.

Measurement of the electric properties of the surface of the skin is preferably performed on the basis of applying a constant DC voltage allowing to measure the electric resistance. Alternatively, an alternating AC voltage with a frequency up to one or more MHz can also be applied allowing an effective measurement of the electrical resistance and even the reactance of the surface of the skin.

According to a further preferred embodiment of the invention, the spectroscopic system further comprises monitoring means for directing a monitoring beam into the volume of interest and for collecting a monitoring return radiation from the volume of interest. This monitoring return radiation is further indicative of a visual image of the volume of interest.

Suitable monitoring or imaging methods, include Orthogonal Polarized Spectral Imaging (OPSI), Confocal Video Microscopy (CVM), Optical Coherence Tomography (OCT), Confocal Laser Scanning Microscopy (CLSM), Doppler Based Imaging and ultrasound based imaging. Corresponding imaging techniques are disclosed US60/262582, EP02732161.1, EP03100689.3 and EP03102481.3.

Typically, the intensity of the monitoring beam is far beneath the intensity of the excitation beam and is therefore not harmful to regions of a body that are generally very sensitive to exposure with radiation, like e.g. eyes.

According to a further preferred embodiment of the invention, the detector element is adapted to generate an intensity signal being indicative of the intensity of the monitoring return radiation. Hence, the intensity signal is indicative of the reflectivity of a subject being in close proximity to the measurement head of the spectroscopic system. Furthermore, this intensity signal is also indicative of the distance between the measurement head and an object.

For example, dropping of the intensity signal below a predefined threshold gives an indication that the measurement head is detached from the surface of the skin. Consequently the means for switching off the excitation beam inhibit propagation of the excitation beam until the intensity of the monitoring return radiation returns within a predefined range. This predefined range covers characteristic values of the intensity of the monitoring beam that gets typically reflected by the surface of the skin of a patient when the measurement head of the spectroscopic system is either in close proximity or even in mechanical contact with the surface of the skin.

Since the spectroscopic system effectively makes use of the monitoring return radiation in order to visualize the volume of interest it already comprises means for detecting the monitoring return radiation, such as e.g. a charge couple device (CCD) camera. In such a case the determination of the intensity of the monitoring return radiation limits to an accumulation of the intensity detected by the single pixels of the CCD array. In contrast to the disclosure of EP 1 080 683 A2 there is no necessity for implementing an additional safety LED and a separate detector that are exclusively designed and implemented for realizing a LED safety device.

According to a further preferred embodiment of the invention, the detector element further comprises an image analysis unit being adapted to recognize the biological structure that can be related to the detection volume. By making use of pattern recognition means, a specific biological structure being relevant for spectroscopic analysis can be effectively traced by the image analysis unit. Pattern recognition can for example be performed on the basis of a visual image of the volume of interest generated by means of the monitoring return radiation. Once a visual image of the volume of interest has been generated and even displayed to a user, the specific biological structure may also be recognized and traced by the user. Hence, the user may also manually perform a pattern recognition. The image analysis unit is further adapted to generate a detection signal for the means for switching on the excitation beam in response of recognition of a biological tubular structure in the visual image.

This allows for releasing of the excitation beam only when a biological structure has been detected in the volume of interest. In particular the recognition of the biological tubular structure is performed prior to a release of the excitation beam. This allows to minimize exposure of radiation to the skin. As a result, exposure can be effectively limited to those regions of the body that are suitable for spectroscopic analysis.

According to a further preferred embodiment of the invention, detection whether the measurement head is in a measurement position can be performed on the basis of the excitation beam. By emitting a low-power excitation beam and detecting corresponding return radiation, a measurement position of the measurement head can be effectively determined. Instead of analyzing the monitoring return radiation, the excitation radiation itself can be analyzed. A monitoring system's functionality for safety precautions can thus be effectively replaced by the means for the spectroscopic analysis. Preferably, the intensity of the return radiation sufficiently indicates whether the excitation beam irradiates into free space or to a surface having e.g. similar light reflecting properties than human skin.

Emitting the excitation beam with low power and/or for a very short time interval effectively prevents damage to light sensitive parts of a human body. After low-power and/or short-time emission of excitation radiation and successive analysis of the return radiation, the accurate spectroscopic measurement step can be performed when correct positioning of the measurement head has been determined. The accurate spectroscopic analysis makes then effective use of a higher power and longer exposure of the excitation beam.

According to a further preferred embodiment of the invention, the output of the pressure sensor is mechanically coupled to the means for switching off the excitation beam. Implementing the means for switching off the excitation beam as a mechanical shutter even allows an all mechanical, robust and precise safety mechanism preventing an accidental release of the excitation beam into free space.

According to a further preferred embodiment of the invention, the spectroscopic system further comprises a beam trap that is arranged opposite to the measurement head. The beam trap and/or the measurement head are designed to form a spectroscopic investigation volume. Preferably, the investigation volume is relatively small allowing only to place a small body part in front of the objective. Larger body parts, like e.g. head, cannot not be placed into the spectroscopic investigation volume. Hence, light-sensitive tissue as e.g. the eyes of a patient cannot become subject to exposure with the excitation radiation. Additionally, the beam trap is non-transparent for the excitation radiation and is highly absorptive. Therefore, irradiation of high-power near infrared laser light into free space can be effectively prevented. In this way, the beam trap serves as an additional means for inhibiting of accidental irradiation of light-sensitive tissue or light-sensitive biological structure. The spectroscopic investigation volume is defined as the volume between beam trap and measurement head of the spectroscopic system, i.e. the volume that is irradiated with high-power laser radiation. In principle, this investigation volume can be arbitrarily implemented by appropriately designing of the beam trap.

In another aspect, the invention provides a computer program product for a spectroscopic system for determining of a property of biological structure in a volume of interest of a patient. The spectroscopic system has a measurement head for directing an excitation beam into the volume of interest. The computer program product comprises computer program means that are adapted to process an output signal obtained from at least one detector element of the measurement head. The computer program means are further adapted to determine if the measurement head is in a measurement position in response of processing of the output signal and to switch the excitation beam in response of processing of the output signal.

In still another aspect, the invention provides a method for determining of a property of a biological structure in a volume of interest of a patient. The inventive method is performed by a spectroscopic system that has a measurement head for directing an excitation beam into the volume of interest. The method comprises the steps of detecting if the measurement head is in a measurement position by making use of at least one detector element and by subsequently switching the excitation beam in response of an output from the at least one detector element.

The invention provides an improved and effective exposure safety mechanism making use of at least one detector element or an arbitrary combination of several detector elements. As described above, the detector element can be implemented as a pressure sensor, a sensor for determining an electrical resistance, a sensor for determining the intensity of a monitoring return radiation or even as an image analysis unit for detecting biological structures within a volume of interest. The inventive method can be based on either one embodiment of the at least one pressure sensor or on an arbitrary combination of various embodiments of a plurality of different detector elements.

For example in a sophisticated embodiment, first a contact pressure is determined by making use of a pressure sensor. Second, the electrical resistance of the subject, e.g. the surface of the skin, being in mechanical contact with the measurement head is determined. After determination of contact pressure and electrical resistance of the subject being in mechanical contact with the measurement head, the monitoring beam is released to the subject and the intensity of the monitoring return radiation is determined in a third step. As a fourth and last step a visual image provided by the monitoring return radiation is analyzed by the image analysis unit in order to recognize any biological structure within the volume of interest. Only in case that all four conditions relating to the parameters: contact pressure, electrical resistance, reflected intensity and recognized biological structures are fulfilled, the excitation beam is released from the measurement head in order to expose a designated capillary vessel underneath the surface of the skin.

Moreover, when the spectroscopic system is in operation mode, i.e. the excitation beam is currently directed into the volume of interest, the inventive protection mechanism permanently checks the fulfillment of any of the above described criteria. Failure of one of the above mentioned conditions may indicate that the measurement head is accidentally detached from the skin. In this case a corresponding signal is transmitted from any one of the at least one detector elements to the switching means that in turn prohibit the propagation of the excitation beam.

It is to be noted, that the present invention is not restricted to a particular type of spectroscopic techniques, as e.g. Raman spectroscopy, but that other optical spectroscopic techniques can also be used. This includes (i) other methods based on Raman scattering including stimulated Raman spectroscopy and coherent anti-Stokes Raman spectroscopy (CARS), (ii) infrared spectroscopy, in particular infrared absorption spectroscopy, Fourier transform infrared (FTIR) spectroscopy and near infrared (NIR) diffusive reflection spectroscopy, (iii) other scattering spectroscopy techniques, in particular fluorescence spectroscopy, multi-photon fluorescence spectroscopy and reflectance spectroscopy, and (iv) other spectroscopic techniques such as photo-acoustic spectroscopy, polarimetry and pump-probe spectroscopy. Preferred spectroscopic techniques for application to the present invention are Raman spectroscopy and fluorescence spectroscopy.

In the following, preferred embodiments of the invention will be described in greater detail by making reference to the drawings in which:
Figure 1 illustrates a block diagram of the inventive protection mechanism making use of a pressure sensor,
Figure 2 illustrates a block diagram of the protection mechanism making use of measuring the electric resistance,
Figure 3 illustrates a block diagram of a sophisticated protection mechanism making use of monitoring return radiation,
Figure 4 shows a flow chart for evaluating release conditions of the excitation beam,
Figure 5 shows a block diagram of a spectroscopic system with a beam trap.

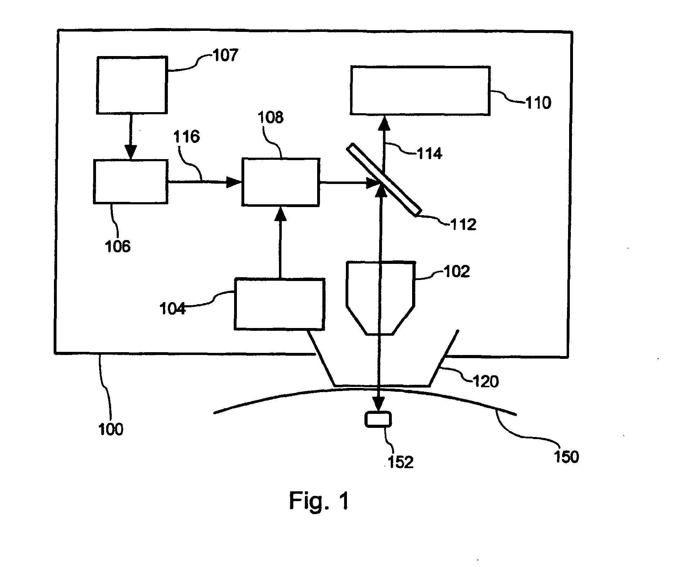

Figure 1 shows a block diagram of the inventive protection mechanism. The spectroscopic system 100 has a laser 106 for generating the excitation beam 116. The spectroscopic system 100 further has a shutter 108, a pressure sensor 104, a measurement head 120, an objective 102, a dichroic mirror 112 a spectrometer 110 and a key module. The spectroscopic system 100 or its measurement head 120 is in close proximity to a skin 150 for spectroscopically analyzing a volume of interest 152 beneath the surface of the skin 150.

The excitation beam 116 emerging from the laser 106 propagates through the shutter 108 that is enabled either to release the laser or to block propagation of the excitation beam 116. When the shutter 108 is open, the excitation beam gets reflected at the dichroic mirror 112 at an angle in order to expose the volume of interest with excitation radiation. Hence, the excitation beam is then directed through the objective 102 focusing the excitation beam 116 into the volume of interest 152, which is e.g. underneath the surface of the skin 150.

The measurement head 120 is substantially transparent for the excitation beam 116 and allows exposure of the volume of interest 152 with radiation. In the illustrated embodiment, the measurement head 120 is mechanically coupled to the pressure sensor 104. For this purpose, the measurement head 120 is elastically supported by the housing of the spectroscopic system 100. When the measurement head 120 is in mechanical contact with the skin 150 thereby exerting a distinct contact pressure between measurement head 120 and skin 150, the pressure sensor 104 generates an output signal that is transmitted to the shutter 108. When the contact pressure and the corresponding output signal of the pressure sensor 104 is within a predefined range that is reasonable to conclude that the spectroscopic system 100 is securely attached to the skin 150, the excitation beam 116 is released by the shutter 108 resulting in an exposure of the volume of interest 152 by the excitation beam.

The key module 107 is adapted to serve as a switch for turning on of the laser 106. It is therefore implemented as a kind of key lock, enabling operation of the laser 106 when an appropriate key is inserted into the key module 107. When access to the key is limited only to authorized personnel, unauthorized usage of the spectroscopic system is effectively prevented. In an even more sophisticated embodiment, the key module may be implemented as a general access control module that enables laser operation only in response of identifying an authorized user. Identification of authorized personnel can be performed by means of checking biometric data of a person, such as fingerprints or by means of an iris scan.

Preferably, the measurement head is designed as a compact hand-held device allowing for an easy and flexible application of the spectroscopic analysis system. Therefore, spectrometer 110, laser 106 as well as shutter 108 are located inside a base station that is connected by fiber optical means with a probe head providing measurement head 120, objective 102 and pressure sensor 104.

Inside the volume of interest, and in particular inside biological tissue being located inside the volume of interest 152, the excitation beam induces various kinds of scattering processes leading to a detectable frequency shift in the spectrum of radiation scattered within the volume of interest. At least a portion of this scattered radiation re-enters the measurement head 120 as return radiation, propagates through the objective 102 and transmits through the dichroic mirror 112 and finally enters the spectrometer 110. The dichroic mirror 112 features a low reflectivity and thus a high transmission for the frequency shifted components of the return radiation. In this way only those parts of the return radiation that were subject of an inelastic scattering process in the volume of interest 152 are transmitted to the spectrometer for spectroscopic analysis allowing to determine a property, as e.g. the composition of blood flowing through a capillary vessel being located within the volume of interest 152.

In this way, the protection mechanism effectively prevents a release of the high power excitation beam 116 when the measurement head 120 is not in an appropriate mechanical contact with an arbitrary object. Since the protection mechanism is also active during exposure of the skin 150 with the excitation beam 116, an accidental detachment of the measurement head 120 from the skin 150 during acquisition of measurement data is effectively detected by the pressure sensor 104 which in turn transmits a signal to the shutter 108 for intercepting the excitation beam 116.

In a rather uncomplicated but precise embodiment, the pressure sensor 104 as well as the shutter 108 can be implemented as all mechanical devices. In this case the pressure sensor 104 has to be mechanically coupled to the shutter 108.

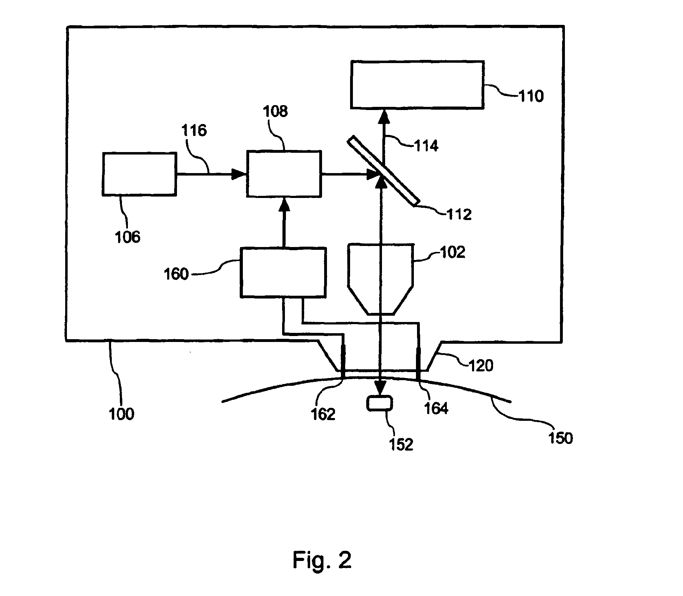

Figure 2 shows a block diagram of the protection mechanism wherein the detector element is implemented as a resistance sensor 160 that is electrically connected to two electrodes 162, 164. All other components of the spectroscopic system 100 almost remain unaltered compared to the embodiment shown in figure 1. The two electrodes 162, 164 are embedded in the measurement head 120. When now the measurement head 120 comes in close proximity to the skin 150 in such a way that the electrodes 162, 164 are in mechanical contact with the skin 150, the resistance sensor 160 is enabled to generate an electrical signal being indicative of the electrical resistance of the skin 150.

Preferably, the resistance sensor 160 applies a small electric voltage to the two electrodes 162, 164 that form an electric circuit in combination with the surface of the skin 150. By measuring the electric current of this electric circuit, the resistance sensor 160 can sufficiently determine the electric resistance between the two electrodes 162, 164. Since the electric resistance of the skin of a patient is certainly lower than for example the resistance of the surrounding air, a mechanical contact between the measurement head 120 and an object having an electric resistance corresponding to the electric resistance of skin can sufficiently be determined. When the electric resistance determined by the resistance sensor 160 is within a range that is typical for the resistance of skin, a corresponding signal is transmitted from the resistance sensor 160 to the shutter 108 for releasing of the excitation beam 116. For determination of the resistance either DC or AC voltage can be applied allowing for measurement of electrical resistance and/or measurement of reactance of the surface of the skin, respectively.

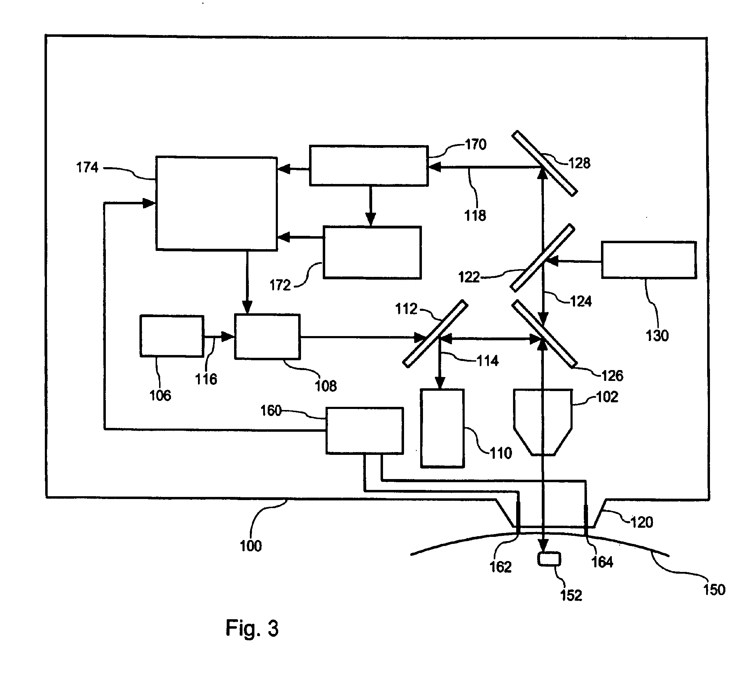

Figure 3 illustrates a block diagram of a sophisticated protection mechanism making use of a monitoring beam for visualization of the volume of interest. In this embodiment the spectroscopic system further has a light source 130 for generating a monitoring beam 124, an imaging unit 170, an image analysis unit 172, a shutter control unit 174 as well as two additional beam splitters 122, 126 and a mirror 128.

The light source 130 emits a monitoring beam 124 that is coaxially aligned with the excitation radiation 116 by reflection at the beam splitter 122. The monitoring beam 124 is transmitted through the dichroic mirror 112 and further through the objective 102 finally exposing the volume of interest 152 for imaging purposes. Typically, the power of the monitoring beam 124 is far beneath the power of the excitation beam 116. At least a portion of the monitoring beam gets reflected in the volume of interest and re-enters the measurement head 120 as well as the objective 102 as monitoring return radiation 118. This monitoring return radiation is partially transmitted through beam splitters 126 and 122. It is finally directed to imaging unit 170 by reflection at the mirror 128.

On the one hand the imaging unit 170 is adapted to generate a visual image from the received monitoring return radiation and on the other hand it is capable to calculate the total intensity of the monitoring return radiation. The magnitude of this total intensity is in turn indicative of the reflectivity of the skin 150 or the volume of interest 152. It can further be exploited to determine whether the monitoring beam 124 gets reflected by some object outside the measurement head 120. Alternatively, the ratio of the intensity of the monitoring beam (124) and the return monitoring beam (118) can be used.

When for example the monitoring beam 124 irradiates into free space due to an accidental detachment of the measurement head 120 from the skin 150, the intensity of the monitoring return radiation 118 determined by the imaging unit 170 will remarkably drop down. In such a case, the imaging unit 170 will transmit a corresponding signal to the shutter control unit 174 finally preventing a release of the excitation beam 116 by means of the shutter 108.

When the measurement head 120 is properly attached to the skin 150, the imaging unit 170 detects an intensity value of the monitoring return radiation 124 that is within a characteristic range. In such a case the imaging unit 170 further processes the received monitoring return radiation 124 and provides an input signal for the image analysis unit 172. The image analysis unit 172 is preferably adapted to perform a pattern recognition for detecting a biological structure, i.e. a capillary vessel, in the visual image derived from the monitoring return radiation. If there is no discernable capillary vessel within the visual image, the image analysis unit 172 transmits a corresponding signal to the shutter control unit 174 for disabling propagation of the excitation beam 116.

In this way the monitoring beam is exploited in a manifold of different ways. First of all it serves to visualize the volume of interest 152 in order to correctly direct the excitation beam into a distinct capillary vessel. Second, the intensity of the monitoring return radiation can be effectively exploited in order to determine whether the measurement head 120 of the spectroscopic system 100 is in close proximity to a non-transparent object. Third, the visual image that is generated on the basis of the monitoring beam can be exploited in order to determine the existence of capillary vessels inside the volume of interest.

In particular, when the intensity of the monitoring return radiation is out of a predefined range or when the image of the monitoring return radiation does not display any kind of capillary vessel, the laser 106 generating the excitation beam is either shut down or the excitation beam 116 is blocked by the shutter 108. Hence the risk of the excitation beam being accidentally directed to sensitive parts of the body is sufficiently minimized.

The embodiment shown in figure 3 also makes use of two electrodes 162, 164 being connected to a resistance sensor 160. The functionality of resistance measurement of the skin is the same as illustrated in figure 2. Here, the resistance sensor 160 is adapted to transmit a signal being indicative of the resistance between the two electrodes 162 and 164 to the shutter control unit 174. By means of the shutter control unit 174 the protection mechanism can be modified according to a plurality of different ways.

Since the imaging unit 170, the image analysis unit 172 as well as the resistance sensor 160 provide signals to the shutter control unit 174 these different signals can be combined by the shutter control unit 174 to operate the shutter 108 according to different levels of protection. In a higher protection level every signal received by the shutter control unit 174 has to be within a predefined range and thus has to indicate a proper attachment of the measurement head 120 and the skin 150. In a lower protection mode for example only one of the received signals has to indicate an attachment of the spectroscopic system 100 and the skin 150.

The illustrated arrangement of beam splitters 122, 126 and dichroic mirror 112 can in principle be replaced by other optical components providing a sufficient direction of the relevant radiation. For example, beam splitter 126 may also be implemented as a dichroic element, providing high reflectivity for the excitation and return radiation and featuring a high transmission for the monitoring radiation.

Generally, the embodiment illustrated in figure 3 indicates only one of a plurality of various examples for implementing an effective protection mechanism. Also here, separation of the spectroscopic system into a base station and a probe head is advantageous. The probe head at least has to provide an objective for directing the excitation radiation into the volume of interest and for collecting corresponding return radiation. Furthermore, the electrodes 162, 164 have to implemented into the probe head. All other components can in principle be located within a base station provided that the optical and electrical signals are sufficiently transmitted between probe head and base station.

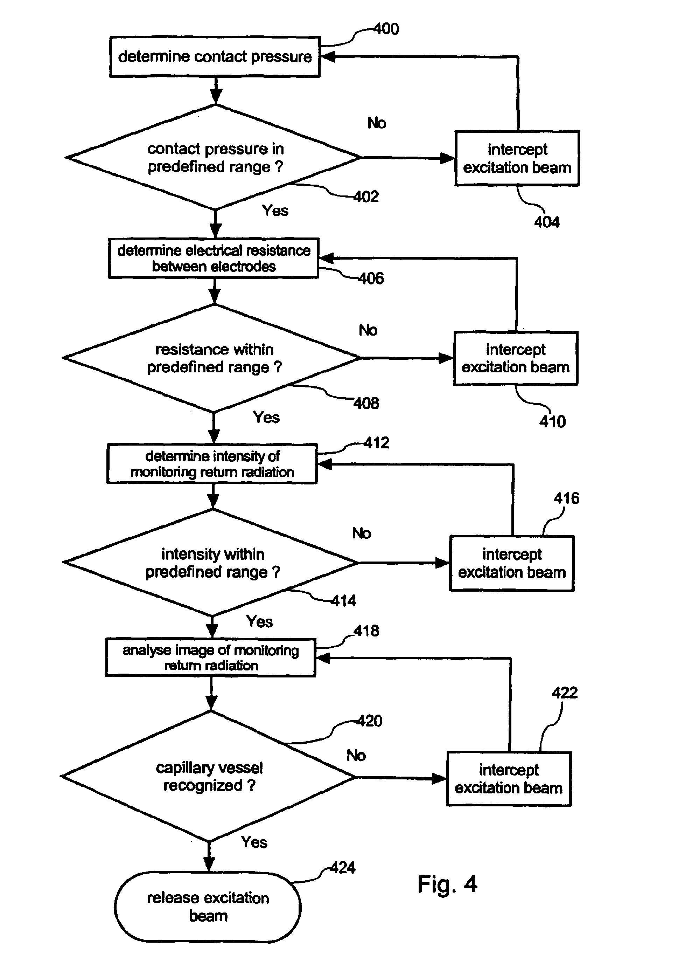

Figure 4 finally illustrates a flow chart for evaluating release conditions of the excitation beam. This flow chart represents a method that is preferably executed by the spectroscopic system and in particular by its shutter control unit for realizing a protection mechanism featuring an high protection level. Here, various types of detector elements provide different parameters being indicative of a proper attachment of the measurement head 120. In a first step 400 a contact pressure is determined by making use of a pressure sensor. In the following step 402 it is checked whether the determined contact pressure is within a predefined range indicating whether the spectroscopic system 100 is in mechanical contact with another object.

If the contact pressure is outside the predefined range, in step 404 the excitation beam is intercepted by means of the shutter or the laser 106 generating the excitation beam is switched off. Thereafter, step 400 is repeatedly performed. The loop consisting of the steps 400, 402, 404 is performed as long as the contact pressure is outside the predefined range.

When in step 402 the contact pressure is within the predefined range, in a following step 406 the electrical resistance between the electrodes is determined. The following step 408 checks whether the determined resistance is within a predefined range being typical for skin. If the determined resistance is outside the characteristic range step 410 is performed and the excitation beam is blocked. Thereafter, the method returns to step 406 for repeatedly determining the electrical resistance between the electrodes. Similar as above, the loop consisting of the steps 406, 408, 410 is performed as long as the electrical resistance between the electrodes of the measurement head is outside the predefined range. Only when in step 408 it is determined that the electrical resistance is within the predefined range step 412 is performed.

In step 412 the monitoring beam is released and the monitoring return radiation is determined. In the following step 414 it is checked whether the intensity of the monitoring return radiation is within a predefined range. If it is outside the predefined range, step 416 is performed leading to an interception of the excitation beam or a switching off of the laser generating the excitation beam. Thereafter step 412 is repeatedly performed. Again, a loop consisting of the steps 412, 414, 416 is repeatedly executed until the intensity of the monitoring return radiation is within a predefined range. In this case step 418 is subsequently performed for analyzing a visual image of the monitoring return radiation.

Analysis of the visual image of the monitoring return radiation as performed in step 418 makes preferable use of pattern recognition means in order to recognize capillary vessels in the visual image. In step 420 it is checked whether any capillary vessel has been recognized in step 418. In case when no capillary vessel has been recognized in step 420, step 422 is subsequently executed leading again to an interception of the excitation beam. Consequently the analysis step 418 is repeatedly executed. The loop consisting of the steps 418, 420, 422 is repeatedly executed until a capillary vessel has been recognized by the analysis of the image of the monitoring return radiation. Only when all four conditions 402, 408, 414 and 420 evaluate to true the excitation beam is finally released in step 424.

A plurality of alternative embodiments for evaluating release conditions of the excitation beam are also conceivable. For example each release condition may only be evaluated once before opening of the shutter and/or turning on of laser operation. In such a case where detachment of the probe head is not to be expected, release conditions are no longer checked during acquisition of spectroscopic data.

However, in a preferred embodiment, each of the release conditions is permanently and independently checked. As soon as only one of the conditions evaluates to false, laser operation stops or emission of the excitation beam is effectively prevented. Any other embodiment comprising a combination of sequential or simultaneous evaluation of release conditions is in principal conceivable.

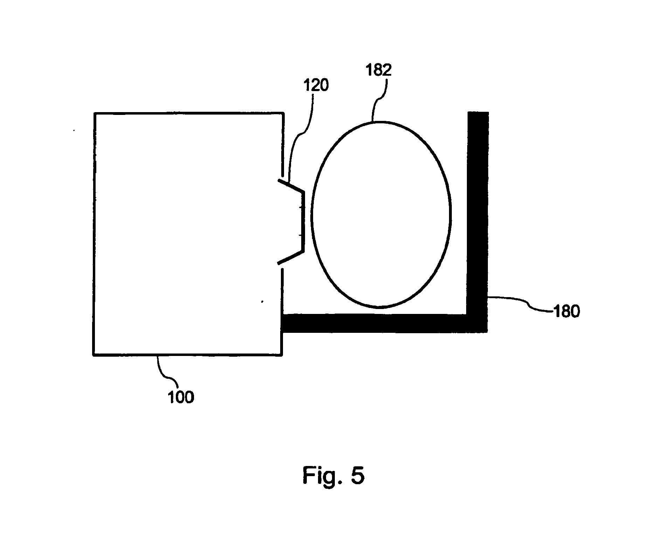

Figure 5 is illustrative of an embodiment of the spectroscopic system 100 wherein the measurement head 120 is covered with a beam trap 180 that is non-transparent and highly absorptive for the excitation wavelength emanating from the measurement head 120. Preferably, the beam trap 180 is designed such that only small parts of a body 182, like e.g. finger, hand, forearm can be placed directly in front of the opening of the measurement head 120. On the one hand, in this way it is effectively prevented that hazardous radiation is irradiated into free space. On the other hand by appropriately designing of the beam trap 180, rather large parts of a body, like e.g. a head cannot be placed directly in front of the measurement head. Hence, the danger of accidentally directing the excitation beam onto light sensitive tissue, as e.g. the eyes of a patient, is effectively minimized.

Moreover, the light beam is effectively inhibited of leaving the volume specified by the beam trap and the housing of the spectroscopic system. Depending on the field of application, various beam traps with a particular geometry may be attached to the housing of the spectroscopic system 100.

The invention provides a plurality of effective means for realizing a safety mechanisms for a spectroscopic system making use of high energetic laser irradiation. The safety mechanism may be implemented as an all mechanical device and/or by making use of a sensor elements that indicate whether the measurement head of the spectroscopic system is in direct contact with the skin of a patient. In this way, hazardous laser irradiation into free space is effectively prevented. By making use of additional image processing means, high-power laser emission can be effectively controlled depending on whether significant biological structures can be identified by pattern recognition means.

### LIST OF REFERENCE NUMERALS

- 100: spectroscopic system
- 102: objective
- 104: pressure sensor
- 106: laser
- 107: key module
- 108: shutter
- 110: spectrometer
- 112: dichroic mirror
- 114: return radiation
- 116: excitation beam
- 118: monitoring return radiation
- 120: measurement head
- 122: beam splitter
- 124: monitoring beam
- 126: beam splitter
- 128: mirror
- 130: light source
- 150: skin
- 152: volume of interest
- 160: resistance sensor
- 162: electrode
- 164: electrode
- 170: imaging unit
- 172: image analysis unit
- 174: shutter control unit
- 180: beam trap
- 182: body part

## Claims

1. A spectroscopic system (100) for determining of a property of a biological structure in a volume of interest (152) of a patient, the spectroscopic system comprising:
- a measurement head (120) for directing an excitation beam (116) into the volume of interest,
- at least one detector element (104; 160; 170, 172) for determining if the measurement head is in a measurement position,
- means for switching (108) the excitation beam in response of an output from the at least one detector element,
wherein the at least one detector element is a pressure sensor (104) being adapted to generate an output being indicative of a contact pressure between the measurement head (120) and the surface of the skin (150),
**characterized in that** said at least one detector element comprises two electrodes (162, 164), the two electrodes sufficiently allow to determine the electric resistance of the surface of the skin when the two electrodes are in mechanical contact with the surface of the skin of a patient, the at least one detector element being adapted to generate an electrical signal being indicative of the electric resistance between the two electrodes.

2. The spectroscopic system (100) according to claim 1, further comprising monitoring means for directing a monitoring beam (124) into the volume of interest (152) and for collecting monitoring return radiation (118) from the volume of interest, the monitoring return radiation being indicative of a visual image of the volume of interest.

3. The spectroscopic system (100) according to claim 2, wherein the detector element (170) being adapted to generate an intensity signal being indicative of the intensity of the monitoring return radiation (118).

4. The spectroscopic system (100) according to claim 2 or 3, wherein the detector element is an image analysis unit (172) being adapted to recognize the biological structure in the visual image of the volume of interest (152) and being further adapted to generate a detection signal for the means for switching (108) the excitation beam in response of recognizing the biological structure in the visual image.

5. The spectroscopic system (100) according to any one of the claims 1 to 4, wherein the means for switching (108) of the excitation beam are adapted to release the excitation beam (116) into the volume of interest (152) only when the contact pressure is within a predefined range.

6. The spectroscopic system (100) according to any one of the claims 1 to 5, wherein the means for switching (108) of the excitation beam are further adapted to release the excitation beam (116) into the volume of interest (152) only when the resistance between the electrodes is within a predefined range.

7. The spectroscopic system (100) according to any one of the claims 3 to 6, wherein the means for switching (108) of the excitation beam (116) are further adapted to release the excitation beam into the volume of interest (152) only when the intensity signal is within a predefined range.

8. The spectroscopic system (100) according to claims 3 to 7, wherein the means for switching (108) of the excitation beam (116) are further adapted to release the excitation beam into the volume of interest (152) only when a detection signal has been generated in response of recognizing the biological structure in the visual image by the image analysis unit (172).

9. The spectroscopic system according to claim 1, wherein the output of the pressure sensor (104) being mechanically coupled to the means for switching (108) the excitation beam, the means for switching being implemented as a mechanical shutter.

10. The spectroscopic system according to claim 1, further comprising a beam trap (180) being arranged opposite to the measurement head (120), the beam trap and the measurement head being designed to form a spectroscopic investigation volume.

11. A computer program product for a spectroscopic system (100) for determining a property of a biological structure in a volume of interest (152) of a patient, the spectroscopic system having a measurement head (120) for directing an excitation beam (116) into the volume of interest, the computer program product comprising computer program means being adapted to:
- process an output signal obtained from at least one detector element (104; 160; 170, 172) of the spectroscopic system,
- determine if the measurement head is in a measurement position in response of processing of the output signal,
- switch the excitation beam in response of processing of the output signal, wherein the at least one detector element is a pressure sensor (104) being adapted to generate an output being indicative of a contact pressure between the measurement head (120) and the surface of the skin (150),
**characterized in that** said at least one detector element comprises two electrodes (162, 164), the two electrodes sufficiently allow to determine the electric resistance of the surface of the skin when the two electrodes are in mechanical contact with the surface of the skin of a patient, the at least one detector element being adapted to generate an electrical signal being indicative of the electric resistance between the two electrodes.

12. The computer program product according to claim 11, wherein the spectroscopic system (100) further comprising monitoring means for directing a monitoring beam (124) into the volume of interest and for collecting a monitoring return radiation (118) from the volume of interest, the monitoring return radiation being indicative of a visual image of the volume of interest (152), the computer program means being further adapted to process the visual image for determining the intensity of the monitoring return radiation.

13. The computer program product according to claim 12, further comprising computer program means being adapted to:
- recognize a biological structure in the visual image,
- generate a detection signal for the means for switching (104; 160; 170, 172) the excitation beam in response of recognizing the biological tubular structure in the visual image.

14. A method for determining of a property of a biological structure in a volume of interest (152) of a patient, the method being performed by a spectroscopic system (100) having a measurement head (120) for directing an excitation beam (116) into the volume of interest, the method comprising the steps of:
- detecting if the measurement head is in a measurement position by making use of at least one detector element (104; 160; 170, 172),
- switching the excitation beam in response of an output from the at least one detector element, wherein the at least one detector element is a pressure sensor (104) being adapted to generate an output being indicative of a contact pressure between the measurement head (120) and the surface of the skin (150),
**characterized in that** said at least one detector element comprises two electrodes (162, 164), the two electrodes sufficiently allow to determine the electric resistance of the surface of the skin when the two electrodes are in mechanical contact with the surface of the skin of a patient, the at least one detector element being adapted to generate an electrical signal being indicative of the electric resistance between the two electrodes.

15. The method according to claim 14, wherein switching of the excitation beam (116) comprising releasing of the excitation beam in response of one or a combination of several of release criteria being fulfilled, the release criteria comprising:
- a contact pressure determined by the at least one detector element being within a predefined range,
- an electrical resistance determined by the at least one detector element being within a predefined range,
- an intensity of the monitoring return radiation of a monitoring beam being within a predefined range,
- a visualized image provided by the monitoring return radiation being indicative of at least one biological structure being within the volume of interest.

## Patentansprüche

1. Spektroskopisches System (100) zur Ermittlung einer Eigenschaft einer biologischen Struktur in einem interessierenden Volumen (152) eines Patienten, wobei das spektroskopische System umfasst:
- einen Messkopf (120), um einen Anregungsstrahl (116) in das interessierende Volumen zu lenken,
- mindestens ein Detektorelement (104; 160; 170, 172), um zu ermitteln, ob sich der Messkopf in einer Messposition befindet,
- Mittel, um den Anregungsstrahl in Reaktion auf ein Ausgangssignal von dem mindestens einen Detektorelement umzuschalten,
wobei das mindestens eine Detektorelement ein Drucksensor (104) ist, der so eingerichtet ist, dass er ein Ausgangssignal erzeugt, das auf einen Kontaktdruck zwischen dem Messkopf (120) und der Oberfläche der Haut (150) schließen lässt,
**dadurch gekennzeichnet, dass** das mindestens eine Detektorelement zwei Elektroden (162, 164) umfasst, wobei die beiden Elektroden es hinreichend ermöglichen, den elektrischen Widerstand der Oberfläche der Haut zu ermitteln, wenn sich die beiden Elektroden in mechanischem Kontakt mit der Oberfläche der Haut eines Patienten befinden, wobei das mindestens eine Detektorelement so eingerichtet ist, dass es ein elektrisches Signal erzeugt, das auf den elektrischen Widerstand zwischen den beiden Elektroden schließen lässt.

2. Spektroskopisches System (100) nach Anspruch 1, das weiterhin Überwachungsmittel umfasst, um einen Überwachungsstrahl (124) in das interessierende Volumen (152) zu lenken und um Überwachungsrückstrahlung (118) von dem interessierenden Volumen aufzufangen, wobei die Überwachungsrückstrahlung ein visuelles Bild des interessierenden Volumens erkennen lässt.

3. Spektroskopisches System (100) nach Anspruch 2, wobei das Detektorelement (170) so eingerichtet ist, dass es ein Intensitätssignal erzeugt, das auf die Intensität der Überwachungsrückstrahlung (118) schließen lässt.

4. Spektroskopisches System (100) nach Anspruch 2 oder 3, wobei das Detektorelement eine Bildanalyseeinheit (172) ist, die so eingerichtet ist, dass sie die biologische Struktur in dem visuellen Bild des interessierenden Volumens (152) erkennt, und weiterhin so eingerichtet ist, dass sie in Reaktion auf das Erkennen der biologischen Struktur in dem visuellen Bild ein Detektionssignal für die Mittel (108) zum Umschalten des Anregungsstrahls erzeugt.

5. Spektroskopisches System (100) nach einem der Ansprüche 1 bis 4, wobei die Mittel (108) um Umschalten des Anregungsstrahls so eingerichtet sind, dass sie den Anregungsstrahl (116) in das interessierende Volumen (152) nur dann freigeben, wenn der Kontaktdruck innerhalb eines vorher definierten Bereichs liegt.

6. Spektroskopisches System (100) nach einem der Ansprüche 1 bis 5, wobei die Mittel (108) zum Umschalten des Anregungsstrahls weiterhin so eingerichtet sind, dass sie den Anregungsstrahl (116) in das interessierende Volumen (152) nur dann freigeben, wenn der Widerstand zwischen den Elektroden innerhalb eines vorher definierten Bereichs liegt.

7. Spektroskopisches System (100) nach einem der Ansprüche 3 bis 6, wobei die Mittel (108) zum Umschalten des Anregungsstrahls (116) weiterhin so eingerichtet sind, dass sie den Anregungsstrahl in das interessierende Volumen (152) nur dann freigeben, wenn das Intensitätssignal innerhalb eines vorher definierten Bereichs liegt.

8. Spektroskopisches System (100) nach einem der Ansprüche 3 bis 7, wobei die Mittel (108) zum Umschalten des Anregungsstrahls (116) weiterhin so eingerichtet sind, dass sie den Anregungsstrahl in das interessierende Volumen (152) nur dann freigeben, wenn in Reaktion auf das Erkennen der biologischen Struktur in dem visuellen Bild durch die Bildanalyseeinheit (172) ein Detektionssignal erzeugt wurde.

9. Spektroskopisches System nach Anspruch 1, wobei der Ausgang des Drucksensors (104) mit den Mitteln (108) zum Umschalten des Anregungsstrahls mechanisch gekoppelt ist, wobei die Mittel zum Umschalten als ein mechanischer Verschluss implementiert sind.

10. Spektroskopisches System (100) nach Anspruch 1, das weiterhin eine Strahlenfalle (180) umfasst, die gegenüber des Messkopfes (120) angeordnet ist, wobei die Strahlenfalle und der Messkopf so ausgeführt sind, dass sie ein spektroskopisches Ermittlungsvolumen bilden.

11. Computerprogrammprodukt für ein spektroskopisches System (100) zur Ermittlung einer Eigenschaft einer biologischen Struktur in einem interessierenden Volumen (152) eines Patienten, wobei das spektroskopische System einen Messkopf (120) umfasst, um einen Anregungsstrahl (116) in das interessierende Volumen zu lenken, wobei das Computerprogrammprodukt Computerprogrammmittel umfasst, die so eingerichtet sind, dass sie:
- ein von mindestens einem Detektorelement (104; 160; 170, 172) des spektroskopischen Systems empfangenes Ausgangssignal verarbeiten,
- in Reaktion auf die Verarbeitung des Ausgangssignals ermitteln, ob sich der Messkopf in einer Messposition befindet,
- den Anregungsstrahl in Reaktion auf die Verarbeitung des Ausgangssignals den Anregungsstrahl umschalten, wobei das mindestens eine Detektorelement ein Drucksensor (104) ist, der so eingerichtet ist, dass er ein Ausgangssignal erzeugt, das auf einen Kontaktdruck zwischen dem Messkopf (120) und der Oberfläche der Haut (150) schließen lässt,
**dadurch gekennzeichnet, dass** das mindestens eine Detektorelement zwei Elektroden (162, 164) umfasst, wobei die beiden Elektroden es hinreichend ermöglichen, den elektrischen Widerstand der Oberfläche der Haut zu ermitteln, wenn sich die beiden Elektroden in mechanischem Kontakt mit der Oberfläche der Haut eines Patienten befinden, wobei das mindestens eine Detektorelement so eingerichtet ist, dass es ein elektrisches Signal erzeugt, das auf den elektrischen Widerstand zwischen den beiden Elektroden schließen lässt.

12. Computerprogrammprodukt nach Anspruch 11, wobei das spektroskopische System (100) weiterhin Überwachungsmittel umfasst, um einen Überwachungsstrahl (124) in das interessierende Volumen zu lenken und um Überwachungsrückstrahlung (118) von dem interessierenden Volumen aufzufangen, wobei die Überwachungsrückstrahlung ein visuelles Bild des interessierenden Volumens (152) erkennen lässt, wobei die Computerprogrammmittel weiterhin so eingerichtet sind, dass sie das visuelle Bild zur Ermittlung der Intensität der Überwachungsrückstrahlung verarbeiten.

13. Computerprogrammprodukt nach Anspruch 12, das weiterhin Computerprogrammmittel umfasst, die so eingerichtet sind, dass sie:
- eine biologische Struktur in dem visuellen Bild erkennen,
- in Reaktion auf das Erkennen der biologischen Struktur in dem visuellen Bild ein Detektionssignal für die Mittel (104; 160; 170, 172) zum Umschalten des Anregungsstrahls erzeugen.

14. Verfahren zur Ermittlung einer Eigenschaft einer biologischen Struktur in einem interessierenden Volumen (152) eines Patienten, wobei das Verfahren, das durch ein spektroskopisches System (100) mit einem Messkopf (120), um einen Anregungsstrahl (116) in das interessierende Volumen zu lenken, ausgeführt wird, die folgenden Schritte umfasst:
- Detektieren, ob sich der Messkopf in einer Messposition befindet, unter Verwendung von mindestens einem Detektorelement (104; 160; 170, 172),
- Umschalten des Anregungsstrahls in Reaktion auf ein Ausgangssignal von dem mindestens einen Detektorelement, wobei das mindestens eine Detektorelement ein Drucksensor (104) ist, der so eingerichtet ist, dass er ein Ausgangssignal erzeugt, das auf einen Kontaktdruck zwischen dem Messkopf (120) und der Oberfläche der Haut (150) schließen lässt,
**dadurch gekennzeichnet, dass** das mindestens eine Detektorelement zwei Elektroden (162, 164) umfasst, wobei die beiden Elektroden es hinreichend ermöglichen, den elektrischen Widerstand der Oberfläche der Haut zu ermitteln, wenn sich die beiden Elektroden in mechanischem Kontakt mit der Oberfläche der Haut eines Patienten befinden, wobei das mindestens eine Detektorelement so eingerichtet ist, dass es ein elektrisches Signal erzeugt, das auf den elektrischen Widerstand zwischen den beiden Elektroden schließen lässt.

15. Verfahren nach Anspruch 14, wobei das Umschalten des Anregungsstrahls die Freigabe des Anregungsstrahls in Reaktion auf das Erfüllen eines oder einer Kombination aus mehreren Freigabekriterien umfasst, wobei sich die Freigabekriterien wie folgt zusammensetzen:
- ein von dem mindestens einen Detektorelement ermittelter Kontaktdruck liegt in einem vorher definierten Bereich,
- ein von dem mindestens einen Detektorelement ermittelter, elektrischer Widerstand liegt in einem vorher definierten Bereich,
- eine Intensität der Überwachungsrückstrahlung eines Überwachungsstrahls liegt in einem vorher definierten Bereich,
- ein durch die Überwachungsrückstrahlung vorgesehenes, visualisiertes Bild lässt auf mindestens eine biologische Struktur in dem interessierenden Volumen schließen.

## Revendications

1. Système spectroscopique (100) permettant de déterminer une propriété d'une structure biologique dans un volume d'intérêt (152) d'un patient, le système spectroscopique comprenant :
- une tête de mesure (120) pour diriger un faisceau d'excitation (116) dans le volume d'intérêt,
- au moins un élément détecteur (104 ; 160 ; 170, 172) permettant de déterminer si la tête de mesure est à une position de mesure,
- un moyen de commutation (108) du faisceau d'excitation en réponse à une sortie de l'au moins un élément détecteur,
dans lequel l'au moins un élément détecteur est un capteur de pression (104) apte à générer une sortie indiquant une pression de contact entre la tête de mesure (120) et la surface de la peau (150),
**caractérisé en ce que** ledit au moins un élément détecteur comprend deux électrodes (162, 164), les deux électrodes permettent suffisamment de déterminer la résistance électrique de la surface de la peau lorsque les deux électrodes sont en contact mécanique avec la surface de la peau d'un patient, l'au moins un élément détecteur étant apte à générer un signal électrique indiquant la résistance électrique entre les deux électrodes.

2. Système spectroscopique (100) selon la revendication 1, comprenant en outre un moyen de surveillance pour diriger un faisceau de surveillance (124) dans le volume d'intérêt (152) et pour collecter le rayonnement de retour de surveillance (118) du volume d'intérêt, le rayonnement de retour de surveillance indiquant une image visuelle du volume d'intérêt.

3. Système spectroscopique (100) selon la revendication 2, dans lequel l'élément détecteur (170) est apte à générer un signal d'intensité indiquant l'intensité du rayonnement de retour de surveillance (118).

4. Système spectroscopique (100) selon la revendication 2 ou 3, dans lequel l'élément détecteur est une unité d'analyse d'image (172) apte à reconnaître la structure biologique dans l'image visuelle du volume d'intérêt (152) et en outre apte à générer un signal de détection pour le moyen de commutation (108) du faisceau d'excitation en réponse à la reconnaissance de la structure biologique dans l'image visuelle.

5. Système spectroscopique (100) selon l'une quelconque des revendications 1 à 4, dans lequel le moyen de commutation (108) du faisceau d'excitation est apte à libérer le faisceau d'excitation (116) dans le volume d'intérêt (152) uniquement lorsque la pression de contact est à l'intérieur d'une plage prédéfinie.

6. Système spectroscopique (100) selon l'une quelconque des revendications 1 à 5, dans lequel le moyen de commutation (108) du faisceau d'excitation est en outre apte à libérer le faisceau d'excitation (116) dans le volume d'intérêt (152) uniquement lorsque la résistance entre les électrodes est à l'intérieur d'une plage prédéfinie.

7. Système spectroscopique (100) selon l'une quelconque des revendications 3 à 6, dans lequel le moyen de commutation (108) du faisceau d'excitation (116) est en outre apte à libérer le faisceau d'excitation dans le volume d'intérêt (152) uniquement lorsque le signal d'intensité est à l'intérieur d'une plage prédéfinie.

8. Système spectroscopique (100) selon l'une quelconque des revendications 3 à 7, dans lequel le moyen de commutation (108) du faisceau d'excitation (116) est en outre apte à libérer le faisceau d'excitation dans le volume d'intérêt (152) uniquement lorsqu'un signal de détection est généré en réponse à la reconnaissance de la structure biologique dans l'image visuelle par l'unité d'analyse d'image (172).

9. Système spectroscopique selon la revendication 1, dans lequel la sortie du capteur de pression (104) est couplée mécaniquement au moyen de commutation (108) du faisceau d'excitation, le moyen de commutation étant mis en oeuvre sous forme d'obturateur mécanique.

10. Système spectroscopique selon la revendication 1, comprenant en outre un piège à faisceau (180) disposé à l'opposé de la tête de mesure (120), le piège à faisceau et la tête de mesure étant conçus pour former un volume d'investigation spectroscopique.

11. Produit de programme informatique pour un système spectroscopique (100) permettant de déterminer une propriété d'une structure biologique dans un volume d'intérêt (152) d'un patient, le système spectroscopique comprenant une tête de mesure (120) pour diriger un faisceau d'excitation (116) dans le volume d'intérêt, le produit de programme informatique comprenant des moyens de programme informatique aptes à :
- traiter un signal de sortie obtenu d'au moins un élément détecteur (104 ; 160 ; 170, 172) du système spectroscopique,
- déterminer si la tête de mesure est à une position de mesure en réponse au traitement du signal de sortie,
- commuter le faisceau d'excitation en réponse au traitement du signal de sortie, dans lequel l'au moins un élément détecteur est un capteur de pression (104) apte à générer une sortie indiquant une pression de contact entre la tête de mesure (120) et la surface de la peau (150),
**caractérisé en ce que** ledit au moins un élément détecteur comprend deux électrodes (162, 164), les deux électrodes permettent suffisamment de déterminer la résistance électrique de la surface de la peau lorsque les deux électrodes sont en contact mécanique avec la surface de la peau d'un patient, l'au moins un élément détecteur étant apte à générer un signal électrique indiquant la résistance électrique entre les deux électrodes.

12. Produit de programme informatique selon la revendication 11, dans lequel le système spectroscopique (100) comprend en outre un moyen de surveillance pour diriger un faisceau de surveillance (124) dans le volume d'intérêt et pour collecter un rayonnement de retour de surveillance (118) du volume d'intérêt, le rayonnement de retour de surveillance indiquant une image visuelle du volume d'intérêt (152), les moyens de programme informatique étant en outre aptes à traiter l'image visuelle pour déterminer l'intensité du rayonnement de retour de surveillance.

13. Produit de programme informatique selon la revendication 12, comprenant en outre des moyens de programme informatique aptes à :
- reconnaître une structure biologique dans l'image visuelle,
- générer un signal de détection pour le moyen de commutation (104 ; 160 ; 170, 172) du faisceau d'excitation en réponse à la reconnaissance de la structure tubulaire biologique dans l'image visuelle.

14. Procédé permettant de déterminer une propriété d'une structure biologique dans un volume d'intérêt (152) d'un patient, le procédé étant effectué par un système spectroscopique (100) comprenant une tête de mesure (120) pour diriger un faisceau d'excitation (116) dans le volume d'intérêt, le procédé comprenant les étapes consistant à :
- détecter si la tête de mesure est à une position de mesure en utilisant au moins un élément détecteur (104 ; 160 ; 170, 172),
- commuter le faisceau d'excitation en réponse à une sortie de l'au moins un élément détecteur, dans lequel l'au moins un élément détecteur est un capteur de pression (104) apte à générer une sortie indiquant une pression de contact entre la tête de mesure (120) et la surface de la peau (150),
**caractérisé en ce que** ledit au moins un élément détecteur comprend deux électrodes (162, 164), les deux électrodes permettent suffisamment de déterminer la résistance électrique de la surface de la peau lorsque les deux électrodes sont en contact mécanique avec la surface de la peau d'un patient, l'au moins un élément détecteur étant apte à générer un signal électrique indiquant la résistance électrique entre les deux électrodes.

15. Procédé selon la revendication 14, dans lequel la commutation du faisceau d'excitation (116) comprend la libération du faisceau d'excitation en réponse à un ou à une combinaison de plusieurs critères de libération étant remplis, les critères de libération comprenant :
- une pression de contact déterminée par l'au moins un élément détecteur est à l'intérieur d'une plage prédéfinie,
- une résistance électrique déterminée par l'au moins un élément détecteur est à l'intérieur d'une plage prédéfinie,
- une intensité du rayonnement de retour de surveillance d'un faisceau de surveillance est à l'intérieur d'une plage prédéfinie,
- une image visualisée fournie par le rayonnement de retour de surveillance indique qu'au moins une structure biologique est à l'intérieur du volume d'intérêt.
